# EUROPEAN PATENT SPECIFICATION

(11) **EP 0 674 909 B1**
(45) Date of publication and mention of the grant of the patent: **20.05.1998**
(21) Application number: 95830096.4
(22) Date of filing: 17.03.1995
(51) Int. Cl.: A61M 1/00, A61B 17/39

(54) **A cannula for fatty tissue aspiration treatment and a handle employing such a cannula**
Kanüle zum Absaugen von Fettgeweben und Handgriff dafür
Canule de liposuccion et poignée pour celle-ci

(30) Priority: 18.03.1994 IT TO940197
(43) Date of publication of application: 04.10.1995
(73) Proprietor: L.I.C.A. di ROSSO & C. S.n.c., I-10040 Caselette (Torino) (IT)
(72) Inventor: Rosso, Luciano, I-10040 Caselette (Torino) (IT)
(74) Representative: Buzzi, Franco

(56) References cited:
- EP-A- 0 543 123
- WO-A-86/05379
- WO-A-93/24066
- US-A- 3 532 095

## Description

The present invention is related in general to cosmetical treatment of the human body by means of removal of masses of adipose tissue, i.e. to fatty tissue aspiration treatments, comprising a fluidizing step of the adipose tissue and a step of extraction of the fluidized adipose tissue, wherein the fluidizing step consists of the combined effect of infiltration of a diluent solution and application of a fluidizing perturbation.

From International Patent Application n. WO 93/24066, in the name of the same Applicant, an apparatus for fatty tissue aspiration treatment is known, wherein the fluidizing perturbation is produced by electro-magnetic waves generated by an emitter through a pair of electrodes which are constituted by two cannulae. According to the above-referenced prior document, the frequency range of the electro-magnetic waves is comprised between 30 Hz and 2 MHz.

Each cannula is constituted by a tubular needle of an electrically conductive material, and is employed either for the supply of the diluent solution or, respectively, for the aspiration of the fluidized adipose tissue, and the needle is provided with a root end connected with a tubular attachment member to a handle. In order to operate as an electrode, each cannula is connected to the electro-magnetic wave generator through a respective outer electrical wire, secured to the needle in proximity to the respective tubular attachment member.

The presence of these outer electrical wires may involve both risks of failures of the apparatus, owing to a possible accidental detachment of the wires relative to the needles of the cannulae, and problems of encumbrance and inconvenience for the operator when using the handle.

The present invention provides an improvement to the system known from the above-referenced document WO 93/24066 and, more particularly, has the object to provide a cannula for fatty tissue aspiration treatment of the above mentioned type, which allows suppression of electrical wires placed outside of the handle for connection thereof to the electro-magnetic wave generator, so as to avoid the above drawbacks while ensuring a constructively simplified design of the fatty tissue aspiration apparatus employing such a cannula.

In order to achieve the above object, the present invention is related to a cannula according to what is set forth in the preamble of claim 1, the primary feature of which resides in that the root end of the needle of the cannula passes through said attachment member and projects therefrom so as to form a plug for wireless direct electrical connection of the electrode.

According to another feature of the invention, the cannula further conveniently comprises a tubular connector of an electrically conductive material defining a socket for axial engagement of said plug root end of the needle.

Moreover, the tubular attachment member of the cannula may to advantage be formed with threaded engagement means.

The invention is also directed to a handle for fatty tissue aspiration treatment apparatus of the type previously specified, which is characterized in that it is provided with a pair of cannulae such as defined in the above, which are parallel and arranged side by side to each other and constitute a pair of electrodes for the emission of electro-magnetic waves, and of which in use one is intended for supplying the diluent solution and the other is intended for aspirating the fluidized adipose tissue.

According to a preferred embodiment, the handle comprises a substantially cylindrical body provided at one end thereof with a pair of innerly threaded axial seats within each of which an outerly threaded bush is fixed, having an inner thread for retaining said threaded engagement means of the tubular attachment member of a respective cannula, each seat housing a conductive ring with which the socket tubular connector of the respective cannula is in electrical contact engagement, and the other end of the handle is provided with a connector electrically connected with the two conductive rings.

Further features and advantages of the invention will become apparent through the following detailed description, with reference to the accompanying drawings purely provided by way of non limiting example, in which:
- figure 1 is a diagrammatic perspective view of a handle for a fatty tissue aspiration treatment apparatus provided with a pair of cannulae according to the invention, and
- figure 2 is a partially sectioned and enlarged view along line II-II of figure 1.

In the drawings, reference numeral 1 generally designates as a whole a handle for a fatty tissue aspiration treatment apparatus according to what is disclosed and illustrated in the above-referenced International Patent application n. WO 93/24066.

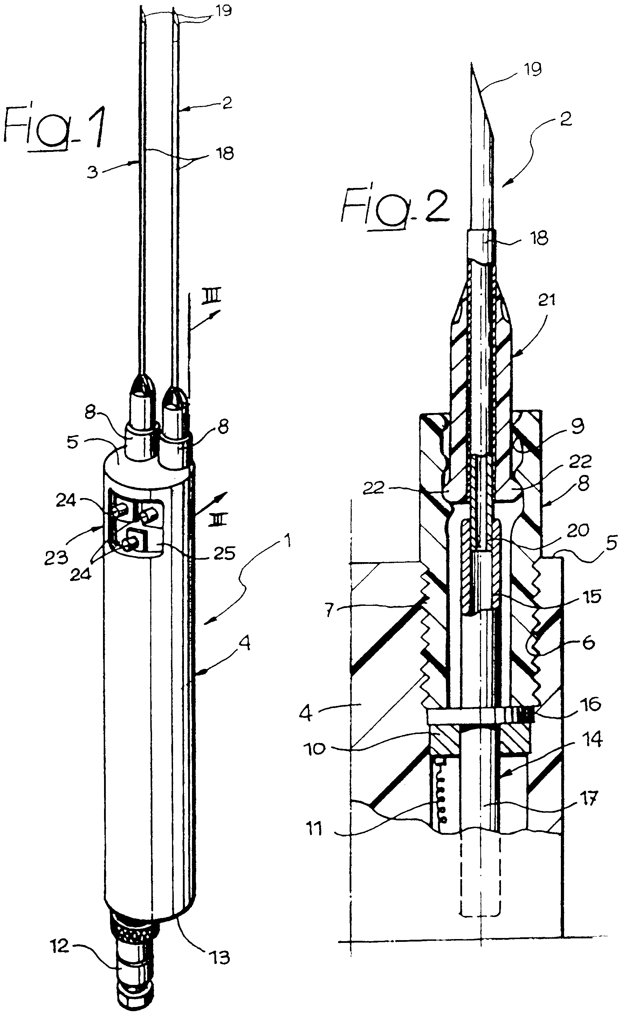

In connection with the present invention, it is sufficient to clarify that the basic elements of the apparatus, connected to the handle 1, comprise a pair of pumps, respectively a delivery (normally peristaltic) pump and a suction pump, intended to supply a fluidizing liquid (for instance distilled water or a physiological solution) and for the extraction of the fluidized adipose tissue relative to the treated area, through a pair of cannulae indicated in figure 1 by reference numbers 2 and 3, respectively.

The cannulae 2 and 3 further operate as electrodes for the application of a fluidizing perturbation into the adipose tissue, which is produced by means of emission of electro-magnetic waves generated by an oscillator circuit. The frequency of the electro-magnetic waves may be comprised in a range between 0,3 and 2 MHz (radio waves), or in a range between 30 and 1000 Hz (low frequency).

According the present invention, the handle 1 is constituted by a substantially cylindrical elongated body 4, normally made of plastic material or any other electrically insulating material, formed with inner passages (not shown) for the connection, in the way clarified herebelow, to a pair of ducts which connect the cannulae 2, 3 with respective outer hoses (not shown), which in turn are connected with the delivery pump of the diluent solution and with the suction pump of the fluidized adipose tissue.

In correspondence of one of its end faces 5, the body 4 is formed with a pair of innerly threaded axial seats, only one of which is shown at 6 in figure 2, into each of which a corresponding outer thread 7 is engaged, formed in a bush 8 of electrically insulating material projecting outwardly of the end face 5.

Each bush 8 is provided in the projecting portion thereof with an inner thread 9, and in correspondence of its inner end is facing towards a conductive ring 10 axially fixed within the seat 6. The two conductive rings 10 are electrically connected, via respective wires 11 housed within the body 4 of the handle 1, to a connector 12 applied onto the other end face 13 of the body 4 for connection to the electro-magnetic wave generator.

In each axial seat 6 of the body 4 a respective tubular connector 14 of electrically conductive material is further fitted, having an outer portion 15 extending within the bush 8, as far as the proximity of the inner thread 9 thereof, and an inner portion 17 passing through the conductive ring 10 and extending for a certain length beneath the latter. The two portions 15 and 17 are separated therebetween by an integral annular collar 16, which is axially blocked on one side against the inner end of the bush 8, and on the other side against the corresponding face of the conductive ring 10. In this way, the tubular connector 14 can be electrically connected, via the ring 10, the respective wire 11 and the connector 12, to the electro-magnetic wave generator.

The portion 17 of the tubular connector 14 functions as an axial connection fitting to one or the other hoses connected to the supply or suction pump, respectively, of the apparatus.

The other portion 15 of each tubular connector 14 constitutes a wireless socket for direct electrical connection of the respective cannula 2, 3.

To such effect, each cannula 2, 3 is designed according to the invention such as shown in detail in figure 2, with specific reference to the cannula 2. The following description is integrally applicable also to the cannula 3.

The cannula 2 is constituted by a needle of electrically conductive material, coated along a substantial area thereof by a layer (normally a double layer) of protective insulating material 18, having a tapered and sharpened free end tip 19 and rigidly connected in proximity to the opposite end, or root end 20 thereof, to a tubular attachment member 22 made of electrically insulating material.

This tubular attachment member 21 is normally of plastic material, and is formed with threaded engagement appendages 22 for screwing thereof within the inner thread 9 of the corresponding bush 8, so as to perform retainment of the cannula 2 relative to the body 4 of the handle 1.

According to a fundamental feature of the invention, the root end 20 of the needle constituting the cannula 2 is protruding outwardly of the tubular attachment member 22, on the side opposite to the tip 19, so as to project axially within the bush 8 to an extent such as to engage the free end of the socket portion 15 of the tubular connector 14. This engagement is automatically accomplished, upon mounting of the cannula 2, following screwing of the engagement appendages 22 of the tubular attachment member 21 within the thread 9 of the bush 8.

In this way, in the mounted condition of the cannula 2 relative to the body 4 of the handle 1, this cannula 2 is electrically connected directly, i.e. without conductive wires outside the handle 1, to the electro-magnetic waves emitter via the tubular connector 14 with the respective collar 16, the ring 10, the inner wire 11 and the connector 12.

To the aim of making more safe and efficient the electrical connection between the plug constituted by the projecting root end 20 of the cannula 2 and the socket defined by the free end of the portion 15 of the tubular connector 14, the plug 20 and the socket 15 may be formed with respective slightly conical surface of mutual axial coupling.

Reference number 23 designates a control assembly of the handle 1, constituted by a triad of electrical push-button switches 24 housed in a lateral seat 25 formed in the body 4 in proximity of its end face 5 from which the cannulae 2 and 3 are projecting. The three switches 24 are operable, during operation of the fatty tissue aspiration apparatus employing the handle 1, to separately control activation and disactivation of the delivery pump of the diluent solution, activation and disactivation of the suction pump of the fluidized adipose tissue, and activation and disactivation of the electro-magnetic wave emitter, respectively.

Naturally the details of construction and the embodiments may be widely varied with respect to what has been disclosed and illustrated, without thereby departing from the scope of the present invention, such as defined in the appended claims.

## Claims

1. A cannula (2, 3) for fatty tissue aspiration treatment comprising a fluidizing step of the adipose tissue and a step of extraction of the fluidized adipose tissue, wherein the fluidizing step consists of the combined effect of the infiltration of a diluent solution and the application of a fluidizing perturbation generated by the emission of electro-magnetic waves through a pair of electrodes, the cannula (2, 3) being constituted by a tubular needle made of an electrically conductive material for the supply of said diluent solution and/or the suction of the fluidized adipose tissue, said needle (2, 3) having a root end (20) connected to a tubular attachment member (21) and acting itself as an electrode, characterized in that the root end (20) of the needle (2, 3) passes through said attachment member (21) and projects therefrom so as to form a connecting plug for wireless direct electrical connection of the electrode (2, 3).

2. Cannula according to claim 1, characterized in that it further comprises a tubular connector made of an electrically conductive material (14) defining a socket (15) for axial engagement therein of said root plug end (20) of the needle (2, 3).

3. Cannula according to claim 1 or claim 2, characterized in that said tubular attachment member (21) is formed with threaded engagement means (22).

4. A handle (1) for fatty tissue aspiration apparatus, characterized in that it is provided with a pair of cannulae (2, 3) according to claims 1 through 3 arranged parallelly and side by side to each other and constituting a pair of electrodes for emission of electro-magnetic waves and of which in use one is intended for supplying the diluent solution and the other is intended for aspirating the fluidized adipose tissue.

5. Handle according to claim 4, characterized in that it comprises a substantially cylindrical body (4) of electrically insulating material provided at one end (5) thereof with a pair of innerly threaded axial seats (6) within each of which an outerly threaded (7) bush (8) is fixed, having an inner thread (9) for retaining said threaded engagement means (22) of the tubular attachment member (21) of a respective cannula (2, 3), each seat (6) housing a conductive ring (10) with which said tubular connector (14) defining said socket (15) of the corresponding cannula (2, 3) is arranged in electrical contact engagement, and in that the other end (13) of the handle (1) is provided with a connector (12) electrically connected with the two conductive rings (10).

6. Handle according to claim 4 or claim 5, characterized in that the body (4) is provided, in proximity of the end (5) thereof formed with said axial seats (6), with a control assembly (23) including a triad of electrical control switches (24).

## Patentansprüche

1. Kanüle (2, 3) für die Fettgewebeabsaugungsbehandlung, die einen Schritt des Fluidisierens des Fettgewebes sowie einen Schritt des Absaugens des fluidisierten Fettgewebes umfaßt, wobei der Schritt des Fluidisierens aus der kombinierten Wirkung des Einleitens einer Verdünnungslösung und des Wirkens einer fluidisierenden Störenergie besteht, die durch das Ausstrahlen elektromagnetischer Wellen über ein Paar Elektroden erzeugt wird, wobei die Kanüle (2, 3) aus einer röhrenförmigen Nadel aus einem elektrisch leitenden Material besteht, die die Verdünnungslösung zuführt und/oder das fluidierte Fettgewebe absaugt, wobei die Nadel (2, 3) ein Fußende (20) hat, das mit einem röhrenförmigen Anbringungselement (21) verbunden ist und selbst als eine Elektrode wirkt, **dadurch gekennzeichnet,** daß das Fußende (20) der Nadel (2, 3) durch das Anbringungselement (21) hindurchtritt und aus selbigem vorsteht, so daß ein Verbindungsstecker für drahtlosen direkten elektrischen Anschluß der Elektrode (2, 3) entsteht.

2. Kanüle nach Anspruch 1, **dadurch gekennzeichnet,** daß sie des weiteren einen röhrenförmigen Verbinder aus einem elektrisch leitenden Material (14) umfaßt, der eine Fassung (15) bildet, in die das Fuß-Steckerende (20) der Nadel (2, 3) axial eingreift.

3. Kanüle nach Anspruch 1 oder Anspruch 2, **dadurch gekennzeichnet,** daß das röhrenförmige Anbringungselement (21) mit einer Gewindeeingriffseinrichtung (22) versehen ist.

4. Griff (1) für eine Fettgewebeabsaugungsvorrichtung, **dadurch gekennzeichnet,** daß er mit einem Paar Kanülen (2, 3) nach den Ansprüchen 1 bis 3 versehen ist, die parallel zueinander und nebeneinander angeordnet sind und ein Paar Elektroden bilden, die elektromagnetische Wellen ausstrahlen, wobei eine von ihnen in Funktion dazu dient, die Verdünnungslösung zuzuführen, und die andere dazu dient, das fluidisierte Fettgewebe abzusaugen.

5. Griff nach Anspruch 4, **dadurch gekennzeichnet,** daß er einen im wesentlichen zylindrischen Körper (4) aus elektrisch isolierendem Material umfaßt, der an einem Ende (5) desselben ein Paar mit Innengewinde versehener axialer Aufnahmen (6) aufweist, in denen jeweils eine mit Außengewinde (7) versehene Buchse (8) befestigt ist, die ein Innengewinde (9) aufweist, um die mit Gewinde versehene Eingriffseinrichtung (22) des röhrenförmigen Anbringungselementes (21) einer entsprechenden Kanüle (2, 3) zu halten, wobei jede Aufnahme (6) einen leitenden Ring (10) aufnimmt, mit dem sich der röhrenförmige Verbinder (14), der die Fassung (15) der entsprechenden Kanüle (2, 3) aufweist, in elektrischem Kontakteingriff ist, und dadurch, daß das andere Ende (13) des Griffs (1) mit einem Verbinder (12) versehen ist, der mit den beiden leitenden Ringen (10) elektrisch verbunden ist.

6. Griff nach Anspruch 4 oder Anspruch 5, **dadurch gekennzeichnet,** daß der Körper (4) in der Nähe des Endes (5) desselben, das mit den axialen Aufnahmen (6) versehen ist, eine Bedienungsbaugruppe (23) aufweist, die drei elektrische Bedienungsschalter (24) enthält.

## Revendications

1. Canule (2, 3) pour un traitement par aspiration des tissus graisseux, comprenant une phase de fluidisation des tissus adipeux et une phase d'extraction des tissus adipeux fluidisés dans lequel la phase de fluidisation consiste dans l'effet combiné de l'infiltration d'une solution diluante et de l'application d'une perturbation fluidisante engendrée par l'émission d'ondes électromagnétiques à travers une paire d'électrodes, la canule (2, 3) étant constituée par une aiguille tubulaire faite d'une matière conductrice de l'électricité et destinée à débiter ladite solution diluante et/ou à aspirer les tissus adipeux fluidisés, ladite aiguille (2, 3) ayant une extrémité côté racine (20) connectée à un élément de fixation tubulaire (21) et jouant elle-même le rôle d'une électrode, caractérisé en ce que l'extrémité côté racine (20) de l'aiguille (2, 3) passe à travers ledit élément de fixation (21) et déborde au-delà de cet élément de manière à former une fiche de connexion pour la connexion électrique directe, sans fil, de l'électrode (2, 3).

2. Canule selon la revendication 1, caractérisée en ce qu'elle comprend en outre un connecteur tubulaire fait d'une matière conductrice de l'électricité (14) qui définit une douille (15) destinée à recevoir par engagement axial ladite extrémité côté racine formant fiche (20) de l'aiguille (2, 3).

3. Canule selon la revendication 1 ou la revendication 2, caractérisée en ce que ledit élément de fixation tubulaire (21) est muni de moyens de prise filetés (22).

4. Poignée (1) pour appareil d'aspiration de tissus graisseux, caractérisée en ce qu'elle est munie d'une paire de canules (2, 3) selon les revendications 1 à 3, disposées parallèlement et côte à côte, qui constituent une paire d'électrodes pour l'émission d'ondes électromagnétiques, et dont, en utilisation, l'une est destinée à débiter la solution diluante et l'autre est destinée à aspirer les tissus adipeux fluidisés.

5. Poignée selon la revendication 4, caractérisée en ce qu'elle comprend un corps (4) sensiblement cylindrique, fait d'une matière isolante de l'électricité, muni, à l'une de ses extrémités (5), d'une paire d'alvéoles axiaux (6) filetés intérieurement, dans chacun desquels est fixé un manchon (8) fileté extérieurement (7), qui présente un filetage intérieur (9) destiné à retenir lesdits moyens de prise filetés (22) de l'élément de fixation tubulaire (21) d'une canule respective (2, 3), chaque alvéole (6) logeant une bague conductrice (10) avec laquelle ledit connecteur tubulaire (14) définissant ladite douille (15) de la canule (2, 3) correspondante est agencé en prise de contact électrique et en ce que l'autre extrémité (13) de la poignée (1) est munie d'un connecteur (12) connecté électriquement aux deux bagues conductrices (10).

6. Poignée selon la revendication 4 ou la revendication 5, caractérisée en ce que le corps (4) est muni, à proximité de son extrémité (5) munie desdits alvéoles axiaux (6), d'un ensemble de commande (23) comprenant un groupe de trois commutateurs électriques de commande (24).
